(19) Europäisches Patentamt
European Patent Office
Office européen des brevets

(11) Numéro de publication: **0 132 164**
**B1**

# (12) FASCICULE DE BREVET EUROPÉEN

(45) Date de publication du fascicule du brevet: **23.09.87**

(51) Int. Cl.[4]: **C 07 K 5/06**, C 07 D 209/20, A 61 K 37/02, A 61 K 31/405

(21) Numéro de dépôt: **84401013.2**

(22) Date de dépôt: **17.05.84**

(54) **Nouveaux dipeptides du L-5-hydroxy-tryptophane, procédés pour leur préparation et médicaments les contenant.**

(30) Priorité: **24.05.83 FR 8308493**

(43) Date de publication de la demande:
**23.01.85 Bulletin 85/4**

(45) Mention de la délivrance du brevet:
**23.09.87 Bulletin 87/39**

(84) Etats contractants désignés:
**BE CH DE GB IT LI LU NL**

(56) Documents cité:
**EP-A-0 056 618**
**CH-A-343 407**
**CH-A-614 702**
**DE-A-2 853 825**

**CHEMICAL ABSTRACTS, volume 61, no. 3, 1964, colonne 2456c, (COLUMBUS, OHIO, US) D. KUPFER "Qualitative method for partial characterization of indole derivatives"**
**CHEMICAL ABSTRACTS, volume 91, no. 17, 1979, page 134, abrégé 134784w (COLUMBUS, OHIO, US) H. TAMIR et al.: "5-Hydroxytryptophyl peptides. Potent inhibitors of a storage compartment of serotonin"**
**CHEMICAL ABSTRACTS, volume 92, no. 1, 1980, page 379, abrégé 376b (COLUMBUS, OHIO, US) H. TAMIR et al.: "Analgesic effects of N-acetyl-5HTP-5HTP amide are not directly related to brain**

(73) Titulaire: **S.A. PANMEDICA Société dite:, Zone Industrielle - Ilot J, F-06510 Carros (FR)**

(72) Inventeur: **Laruelle, Claude, Avenue Bellevue, F-06270 Villeneuve- Loubet (FR)**
Inventeur: **Lepant, Marcel, L'Escoundu Allée Clairefontaine, F-06140 Vence (FR)**
Inventeur: **Raynier, Bernard, 9 Chemin du Malvan, F-06800 Cagnes (FR)**

(74) Mandataire: **Orès, Bernard, Cabinet ORES 6, Avenue de Messine, F-75008 Paris (FR)**

(56) Documents cité: (suite)
**serotonin levels"**

**Le dossier contient des informations techniques présentées postérieurement au dépôt de la demande et ne figurant pas dans le présent fascicule.**

EP 0 132 164 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention est relative à de nouveaux dipeptides du -L-5-hydroxy-tryptophane, à leurs procédés de préparation et à des médicaments contenant lesdits dérivés.

On connaît le rôle particulièrement important joué par la sérotonine en tant que neuro-médiateur et on a largement étudié son influence dans le mécanisme du sommeil et dans certains types de dépression nerveuse. La Demanderesse qui a une grande expérience dans le domaine des dérivés de la sérotonine (cf. notamment le Brevet français 2 499 076) a contribué à mettre en évidence des interactions importantes entre le système sérotoninergique et les autres systèmes cholinergique ou dopaminergique. Tout dérèglement du système sérotoninergique entraîne donc des conséquences directes comme la dépression ou indirectes par répercussion sur les taux synaptiques des autres amines endogènes, la sérotonine semblant impliquée dans certains syndrômes de Parkinsonisme ou dans la maladie d'Alzheimer. Ce dérèglement peut être corrigé par administration de -L-hydroxy-5 tryptophane (-L-5-HTP), précurseur direct de la sérotonine (5-HT). On sait cependant que l'administration par voie orale de -L-5-HTP ne permet qu'une faible augmentation de son taux cérébral (cf.le B.F.2 499 076 déjà cité). Un franchissement de la barrière hémato-encéphalique assez médiocre et une décarboxylation périphérique importante obligent donc à administrer des quantités élevées de -L-5-HTP.

La présente invention s'est fixé pour but de pourvoir à une nouvelle famille de dérivés de -L-5-HTP qui permettent de corriger les teneurs cérébrales en 5-HTP et en 5-HT à des doses relativement faibles permises par un excellent passage des barrières digestives et hémato-encéphaliques et aussi par une décarboxylation périphérique peu élevée. Leurs toxicités étant d'autre part plus faibles que celle du -L-5-HTP, l'index thérapeutique au plan de l'activité sérotoninergique s'en trouve considérablement augmenté.

La présente invention a pour objet des dipeptides de -L-5-hydroxy-tryptophane de formule générale I ci-après:

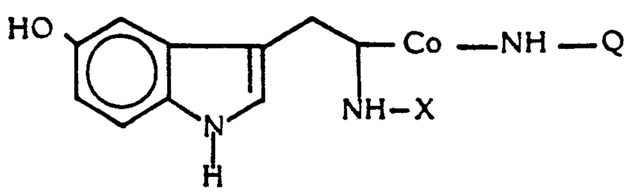

(I)

dans laquelle:

X représente l'hydrogène ou un radical acyle inférieur,

Q représente un reste d'amino-acide libre à l'exception de la tyrosine, ou estérifié, y compris la tyrosine,

ainsi que leurs sels d'addition acides ou basiques pharmaceutiquement compatibles.

La présente invention a également pour objet un procédé de préparation des dipeptides conformes à la présente invention, caractérisé en ce que:

a) l'on prépare un dérivé bloqué sur l'azote en α du L-5-hydroxy-tryptophane,

b) l'on prépare un dérivé bloqué d'un acide aminé en ne laissant subsister que le groupe -NH comme fonction réactive,

c) l'on condense les deux produits bloqués en présence d'un agent de condensation dans un solvant approprié à une température comprise entre 0 et 30° C,

d) l'on débloque éventuellement les fonctions protégées.

Selon un mode de réalisation avantageux du procédé objet de la présente invention, les groupes du blocage sur l'azote en α du -L-5-hydroxy-tryptophane (5-HTP) sont choisis parmi les radicaux carbobenzyloxy-, para-méthoxy-benzyloxy-carbonyle, terbutyloxycarbonyle, les sels de DANE (DANE.E Angew.Chem. Int. Ed.1962,1,658) avec l'acétylacétone ou l'acétoacétate d'éthyle.

Selon un autre mode de réalisation avantageux du procédé objet de la présente invention, le blocage de la ou des deux fonctions acide de l'acide aminé est effectué à l'aide de composés choisis dans le groupe qui comprend les esters d'alcools primaires, secondaires ou tertiaires, les esters benzyliques.

Selon un mode de réalisation particulièrement avantageux du procédé objet de la présente invention, le-L-5-hydroxy-tryptophane bloqué sur l'azote en α est utilisé sous forme d'un ester réactif, ledit ester étant choisi parmi les esters cyanométhyliques, 4-nitrophényliques, pentachlorophényliques.

Conformément à l'invention, la condensation des produits bloqués est effectuée en présence du dicyclohexylcarbodiimide dans un solvant, pris dans le groupe qui comprend le diméthylformamide, le diméthylsulfoxyde, la pyridine, le chloroforme, le dichlorométhane, le tétrahydrofuranne et l'acétone.

Egalement conformément à l'invention, le déblocage des fonctions protégées est effectué par l'hydrogénation catalytique ou l'hydrolyse ménagée.

Outre les dispositions qui précèdent, l'invention comprend encore d'autres dispositions, qui ressortiront de la description qui va suivre.

La présente invention pourra être mieux comprise à l'aide du complément de description qui va suivre, dans lequel on trouvera des exemples de mises en oeuvre du procédé de préparation des nouveaux dérivés conformes à l'invention ainsi qu'un compte-rendu d'expérimentation pharmacologique.

Il doit être bien entendu toutefois que ces exemples et compte-rendu sont donnés uniquement à titre d'illustration, mais n'en constituent en aucune manière une limitation.

Il est à noter que tous les produits ont été soumis à l'analyse par chromatographie en couche mince et ne présentent qu'un seul spot.

Les chromatographies sur couche mince (CCM) ont été réalisées sur plaque Kieselgel F 254 et développées dans les systèmes suivants:

A - butanol 8 - acide acétique 1 - eau 1

B - acétonitrile 85 - eau 10 - acide acétique 5

et révélées en lumière ultra-violette à 254 nm.

Les résultats des analyses élémentaires pratiquées sur tous les produits sont conformes aux formules théoriques.

**Exemples de preparation**

**Exemple 1 - Hydroxy-5-L-tryptophyl-L-aspartate de diéthyle**

a) N-benzyloxycarbonyl-benzyloxycarbonyloxy-5-L-tryptophyl-L-aspartate de diéthyle

Dans un appareil de 2 litres convenablement équipé, on ajoute sous azote 34,16 g (70 mMoles) de -L-N-O bis benzyloxycarbonyl hydroxy-5 tryptophane, préparé selon le B.F. 2 499 076, à 500 ml de chloroforme sec pur sans éthanol. On ajoute rapidement l'aspartate de diéthyle obtenu en mélangeant dans 200 ml de chloroforme pur 15,60 g (70 mMoles) de chlorhydrate de -L-diéthyle aspartate et 7,44 g (73,5 mMoles) de triéthylamine. A la solution obtenue et refroidie vers +5°C, on ajoute lentement en 45 mn 15,90 g (77 mMoles) de dicyclohexyl carbodiimide en solution dans 100 ml de chloroforme pur. On agite ensuite à température ambiante. La réaction dont on peut suivre l'avancement par CCM est complète en 46 heures. Après avoir évaporé le solvant, on reprend le produit par l'acétone qui permet de séparer la dicyclohexylurée. On évapore à nouveau le solvant, on reprend au chloroforme, on lave à l'eau, puis avec une solution diluée de bicarbonate de sodium, ensuite avec une solution diluée d'acide chlorhydrique et enfin à l'eau. La couche chloroformique après séchage sur sulfate de sodium anhydre est diluée à l'éther éthylique.

Après quelques jours à +5° la solution laisse cristalliser le N-O bis benzyloxycarbonyl (hydroxy-5) tryptophyl-aspartate de diéthyle (24,30 g): (rendement: 52,7 %). Le produit présente un seul spot de Rf = 0,9 en CCM sur plaque de silice dans le système chloroforme 1:acétone 1.

b) Hydroxy-5-L-tryptophyl-L-aspartate de diéthyle

On hydrogène sous pression atmosphérique à 40-50°C 24 g (36 mMoles) de produit précédemment obtenu en solution dans 100 cc d'acide acétique et 300 cc d'éthanol en présence de 2 g de palladium à 5 % sur charbon. Après filtration du catalyseur, on évapore le solvant et on reprend par l'éther éthylique qui laisse cristalliser 9,8 g (25 mMoles). On peut recristalliser dans 50 volumes de cyclohexane pour obtenir un produit pur présentant les caractéristiques analytiques suivantes:

- CCM sur plaque Kieselgel dans le système A butanol 8, acide acétique 1, eau 1, un seul spot de Rf 0,65.
- Spectre de RMN en solution dans $CD_3CN$ par rapport au TMS:

à 8 ppm (m) large $NH_2$; 7,2 à 6,5 ppm (m) 4 H (arom); 4,7 ppm (m) (1 H)-($NH_2$)-$\underline{CH}$-CONH; 4,3 à 3,8 ppm 2 (q) 4 H 2 $\underline{OCH_2}CH_3$;

3,6 ppm (m) 1 H, CONH$\underline{CH}$COO; 3 ppm (m) (2 H) $\underline{CH_2}$CH ($NH_2$)-CO;

2,7 ppm (m) (2 H) $\underline{CH_2}$COO; 1,15 (t) (6 H) 2-$OCH_2\underline{CH_3}$.

- Spectre infra-rouge (KBr): 3300$^{cm-1}$ $NH_2$; 1740$^{cm-1}$ (FF) ester;

1650$^{cm-1}$ (FF) amide; 1550$^{cm-1}$ (F) amide: 1200/1250$^{cm-1}$ ester.

c) -L-(hydroxy-5) tryptophyl-L-aspartate de diéthyle, chlorhydrate

On disperse dans l'eau 3,70 g (9,5 mMoles) du produit obtenu précédemment selon b) et on ajoute 9,5 ml d'acide chlorhydrique normal, on traite la solution au charbon actif, puis on lyophilise la solution. On obtient un solide amorphe de PF peu net entre 90 et 100° et dont le spectre de RMN enregistré dans $D_2O$ présente les mêmes signaux caractéristiques que la base obtenue selon b).

**Exemple 2 - Acide (hydroxy-5-L-tryptophyl)-L-aspartique**

a) N-benzyloxycarbonyl-hydroxy-5-L-tryptophanyl-aspartate de dibenzyle

On ajoute 58,2 g (0,12 mMoles) de paratoluène sulfonate de -L-aspartate de dibenzyle préparé selon A.K.BOSE et R.E.STRUBE [J.of Pharm. Sc 9, 847 (1963)] à 300 ml de chloroforme rigoureusement pur, on abaisse la température du milieu vers + 8°C et on ajoute 17 ml de triéthylamine. Après environ 30 minutes, on ajoute 43,5 g (0,12 moles) de -L-N-[benzyloxycarbonyl] (hydroxy-5) tryptophane (préparé par exemple selon le B.F. 2 499 076) dissous dans 800 ml de chloroforme. On ajoute ensuite 24,8 g (0,12 Mole) de dicyclohexyl carbodiimide en solution dans 100 ml de chloroforme. Après 48 heures d'agitation à température ordinaire, on filtre l'insoluble, on évapore le filtrat puis on reprend deux fois de suite par l'acétone pour séparer les traces de

dicylohexylurée. Après reprise à l'éther, on obtient un produit pur de pF = 177°C et $|\alpha|_D$ = -22,3° (C=1, acétone). La CCM sur Kieselgel dans le système toluène 10 formiate d'éthyle 10 acide formique 1 donne un seul spot de Rf = 0,70.

b) Acide hydroxy-5-L-tryptophyl-L-aspartique, trihydrate

On dissout 32 g (0,05 Mole) de dérivé précédent obtenu selon a) dans 600 ml d'acide acétique et 600 ml d'éthanol et hydrogène à pression atmosphérique en présence de 3 g de palladium à 5 % sur charbon. A la fin d'absorption d'hydrogène, on filtre, on évapore, on réempâte à l'éther et on recristallise dans l'eau. On obtient ainsi 86 % du dérivé du titre, trihydrate de pF 190°C et $|\alpha|_D$ = + 12,40°C (C=1, $NH_4OH$ 0,1 N). La CCM du produit sur plaque Kieselgel dans le système: isopropanol 2, acétate d'éthyle 2, eau 1, acide acétique 0,1 donne un seul spot de Rf = 0,65.

**Exemple 3 - Hydroxy-5-L-tryptophyl-L-glutamate de diéthyle, chlorhydrate**

a) N-benzyloxycarbonyl hydroxy-5-L-tryptophyl-L-glutamate de diéthyle

On ajoute à 0°C 33,5 g (0,14 Mole) de chlorhydrate de glutamate de diéthyle préparé selon Brenner (Helv. 36 1109; (1053)) à 300 ml de chloroforme pur et on coule goutte-à-goutte 19,5 cc de triéthylamine. On ajoute ensuite 50 g (0,14 Mole) de -L-N-[benzyloxycarbonyl] (hydroxy-5) tryptophane (préparé selon le B.F. 2 499 076) et on ajoute lentement 29 g de dicyclohexyl-carbodiimide en solution dans 100 ml de chloroforme. On agite 24 heures à température ordinaire puis on filtre l'urée et on évapore à sec. On reprend encore deux fois par 200 ml d'acétone pour éliminer par filtration la dicyclohexylurée encore présente. On évapore, on lave à l'eau par empâtage, puis on recristallise le produit brut dans l'acétate d'éthyle; on obtient ainsi le -L-[N-benzyloxycarbonyl (hydroxy-5) tryptophyl]-L-glutamate de diéthyle pur de pF = 154/5°C avec un rendement de 69 %. Le produit donne un seul spot en CCM sur Kieselgel dans le système A butanol 8, acide acétique 1, eau 1 de Rf = 0,95. $|\alpha|_D$ = -28° (C=1, acétone).

b) (Hydroxy-5-L-tryptophyl)-L-glutamate de diéthyle, chlorhydrate

On hydrogène 50 g (93 millimoles) du produit précédent dans 250 ml d'acide acétique en présence de 3 g de palladium à 5 % sur charbon. Après évaporation du solvant, on reprend à l'eau, on neutralise à l'ammoniaque diluée jusqu'à pH 7,0 et on extrait à l'acétate d'éthyle. Après évaporation du solvant, on reprend par 170 ml d'éthanol et on ajoute 21 ml d'une solution 4 M d'acide chlorhydrique dans l'éthanol. On évapore ensuite à basse température et on concrétise à l'éther. Le produit présente une seule tache en CCM sur Kieselgel dans le système A butanol 8, acide acétique 1, eau 1, de Rf = 0,60.

$|\alpha|_D$ = + 2,9° (C=1, eau). Le spectre de RMN en solution dans $D_2O$ présente les caractéristiques suivantes: 7,5 à 6,7 ppm (m) (arom) (4 H); 4,1 (ppm) 2 (q) (4 H) $OCH_2CH_3$; 3,2 ppm (d) (2 H) $CH_2$ $CH(NH_3)$; (+); 2,0 ppm (m) (4 H) $CH_2$-$CH_2$; 1,10 ppm 2 (t) (6 H) 2-$OCH_2CH_3$. La spectrométrie IR dans le KBr présente les absorptions suivantes: 3500 à 2500$^{cm-1}$ -massif large COOH et $NH_3$+; 1730$^{cm-1}$ ester (FF); 1670$^{cm-1}$ amide (F); 1200$^{cm-1}$ (ester).

**Exemple 4 - Acide hydroxy-5-L-tryptophyl-L-glutamate**

a) [-L-benzyloxycarbonyl (hydroxy-5) tryptophyl]-L-glutamate de dibenzyle

On met en suspension 32,5 g (65 millimoles) de paratoluène sulfonate de -L-dibenzyl-glutamate préparé selon A.K.BOSE et R.E.STRUBE (J.of Pharm.Sc.9, 647 (1983)) dans 300 ml de chloroforme pur, on ajoute 65 millimoles de triéthylamine, on agite 30 mn puis on ajoute 23 g (65 millimoles) de -L-N-benzyloxycarbonyl hydroxy-5 tryptophane préparé selon le B.F. 2 499 076, dissous dans 50 ml de chloroforme pur et 50 ml d'acétone pure. On coule enfin lentement 13,4 g (65 millimoles) de dicyclohexylcarbodiimide en solution dans 50 ml de chloroforme. Après quelques heures, la réaction est complète, on dilue largement à l'acétone pour précipiter complètement la dicyclohexylurée. On évapore la solution et on reprend par l'acétate d'éthyle qu'on lave à l'eau légèrement acide, à la soude diluée puis enfin à l'eau. Après séchage sur sulfate de sodium sec, on évapore, on recristallise dans l'isopropanol et on obtient 80 % de -L-N-[benzyloxycarbonyl-(hydroxy-5 tryptophyl]-L-glutamate de dibenzyle pur de pF = 150°C d' $|\alpha|_D$ = -25,5° (C=1, acétone) présentant en CCM sur Kieselgel dans le système toluène 10, formiate d'éthyle 10, acide formique 1, un seul spot de Rf = 0,50.

Le produit présente d'autre part les caractéristiques spectrales suivantes:

RMN en solution dans $CDCl_3$ par rapport au TMS; 8,0 ppm (s) (1 H) OH phénol; 7,90 ppm (s) (5 H) (ar) benzyl; 7,2 à 6,5 ppm (m) (4 H) arom; 5,7 et 5,5 ppm 2 (m) (2 H) N-H; 5,0 ppm 2 (s) (6 H) $CH_2$-Ar; 4,6 ppm (m) (2 H) CH (NH) CO; 3,2 ppm (m) 2 H Ar-$CH_2$-CH-CO; 2,1 ppm (m) (4 H)-$CH_2$-$CH_2$. I.R. dans KBr: 3450 et 3300$^{cm-1}$ (F)-NH; 1735$^{cm-1}$ (FF) ester; 1690$^{cm-1}$ (F):

1655$^{cm-1}$ et 1530$^{cm-1}$ (FF) amide: 1215$^{cm-1}$ ester.

b) Acide hydroxy-5-L-tryptophyl-L-glutamique

On hydrogène 25 g (36 millimoles) du produit précédent dans 200 ml d'acide acétique en présence de 2,5 g de palladium à 5 % sur charbon. On évapore à sec, on reprend d'abord à l'acétate d'éthyle puis on recristallise dans 20 volumes d'eau; on obtient 62 % d'acide -L-(hydroxy)-5 tryptophyl -L-glutamique de pF 230/2°C titrant 99,7 %.

$|\alpha|_D$ = +17,6° (C=1, ammoniaque N/10) et présentant en spectrométrie RMN dans l'acide trifuoroacétique les caractéristiques suivantes:

7,5 à 6,5 ppm (m) (5 H) arom; 4,5 ppm (m) (2 H) CH (NH) CO;

3,25 ppm (m) (2 H) $ArCH_2CHCO$ 2,3 à 2,0 ppm (m) (4 H) $CH_2$-$CH_2$.

**Exemple 5 - Hydroxy-5-L-tryptophyl-L-tyrosine ester méthylique, chlorhydrate**

a) Ester pentachlorophénylique du N-benzyloxycarbonyl hydroxy-5-L-tryptophane

On ajoute 115 g de -L-N-benzyloxycarbonyl hydroxy-5 tryptophane, préparé selon le B.F. 2 499 076 et 86 g (0,325 mole) de pentachlorophénol dans 1,2 litres de tétrahydrofuranne, on ajoute ensuite 67 g (0,325 mole) de dicyclohexylcarbodiimide dissous dans 200 ml de tétrahydrofuranne.

Après 20 heures d'agitation à température ordinaire, on filtre la dicyclohexylurée, on évapore le filtrat, on reprend à l'acétate d'éthyle qu'on lave successivement à l'acide chlorhydrique 1 N, à la soude 0,1 N et à l'eau, après séchage sur sulfate de sodium on évapore à sec sous pression réduite. Le résidu est repris à l'éther de pétrole et l'on obtient ainsi le dérivé du titre sous forme de cristaux de pF 135°/140°C avec un rendement de 80 %.

b) N-benzyloxycarbonyl hydroxy-5-L-tryptophyl-L-tyrosine, ester méthylique

On ajoute 9,3 g (0,04 mole) de chlorhydrate de l'ester méthylique de la -L-tyrosine à 50 ml de diméthylformamide, puis à 0°C on ajoute 0,04 mole de triéthylamine et 24 g (0,04 mole) de N-benzyloxycarbonyl hydroxy-5 tryptophane (-PCP ester) selon a) dissous dans 50 ml de diméthylformamide. On laisse revenir à température ambiante et on agite pendant 20 heures. On coule ensuite dans un excès d'eau froide. On reprend le précipité par l'acétate d'éthyle qu'on lave à l'acide chlorhydrique normal, à la soude décinormale, puis à l'eau. Après séchage sur sulfate de sodium, la solution est évaporée à sec et le produit est chromatographié sur colonne de silice dans le système chloroforme 3, acétone 1. On obtient 42 % de produit de pF 130/3°C et $|\alpha|_D$ = -18,5° (C=1, acétone) présentant les caractéristiques spectrales suivantes en RMN (DMSOd6):

- 10 ppm (s) (1 H) OH phénol; 9 ppm (s) (1 H) OH phénol; 8 ppm (m) (2 H)-NH-; 7,2 ppm (s) (5 H) phényl; 7,0 à 6,7 ppm (m) (9 H) arom; 4,6 ppm (s) (2 H) $CH_2$-benzyl-; 4,3 ppm (m) (2 H) -CH (NH)-CO; 3,5 ppm (s) (3 H) $OCH_3$; 2,8 ppm (m) (4 H) -$CH_2$-.

en infra-rouge (KBr): 3400-3300$^{cm-1}$ OH et NH; 1730$^{cm-1}$ ester; 1680$^{cm-1}$ NH CO-$OCH_2$; 1650 et 1550$^{cm-1}$ CONH; 1220$^{cm-1}$ ester.

c) Hydroxy-5-L-tryptophyl-L-tyrosine ester méthylique, chlorhydrate

On hydrogène le produit obtenu précédemment dans 300 ml d'éthanol et 1 ml d'acide acétique en présence de 1 g de palladium à 5 % sur charbon. Après filtration, on évapore sous pression réduite puis on reprend à l'eau additionnée d'acide chlorhydrique. On traite au charbon actif et on lyophilise. On obtient 90 % de cristaux beiges titrant 97 % en anhydrotitrimétrie et d' $|\alpha|_D$ -11,6° (C=1, eau).

**Exemple 6 - N-acétyl hydroxy-5-L-tryptophyl-L-tyrosine, ester méthylique**

a) N-acétyl hydroxy-5-L-tryptophane

On refroidit vers +5°C la suspension de 88 g (0,4 mole) de -L-5 HTP dans 400 ml d'eau et on coule simultanément 61 ml d'anhydride acétique et une solution de soude diluée pour maintenir le pH entre 7 et 7,5. La réaction est complète après deux heures, on refroidit à 0°C, on acidifie lentement par HCl dilué. Le dérivé -L-N acétyl (acétyloxy-5) tryptophane précipité est filtré, séché. pF 167/169°C. On l'ajoute à raison de 15 % environ à une solution éthanolique ammoniacale saturée. Après une nuit d'agitation à température ordinaire, il apparaît un précipité blanc qu'on filtre et lave. Ce précipité est mis en solution aqueuse, celle-ci, après acidification laisse cristalliser le -L-N- acétyl (hydroxy-5) tryptophane qu'on peut recristalliser dans 5 volumes d'eau: pF = 209°C $|\alpha|_D$= +13,4° (C=1, ammoniaque 0,1 N)

-13,1° (C=1, éthanol)

Le titre acidimétrique est de 99 %. Les caractéristiques spectrales sont les suivantes: en RMN (dans $D_2O$): 7,2 ppm (d) (1 H) J = 8 Hz 1 H; 7,10 ppm (s) (1 H); 7,0 ppm (1 H) (d) J' = 2 Hz; 6,70 ppm dd (1 H); 4,5 ppm (m) (1 H) CH (NH)CO; 3,1 ppm (m) (2 H) -$CH_2$-; 1,8 ppm (s) (3 H) $CH_3CO$; en infrarouge (dans KBr): 3440, 3440, 3320$^{cm-1}$ NH, OH; 2500 à 1800$^{cm-1}$ massif COOH; 1690$^{cm-1}$ COOH - 1630 et 1550$^{cm-1}$ -CONHR.

b) N-acétyl hydroxy-5-L-tryptophyl-L-tyrosine, ester méthylique

On dissout 14 g (60 mMoles) de chlorhydrate de l'ester méthylique de la -L-tyrosine dans 30 ml d'eau, on libère la base par addition de $CO_3HNa$ et on extrait par l'acétate d'éthyle la base tyrosine ester qui cristallise au froid.

Après filtration et séchage, on mélange 46 millimoles de base-tyrosine ester à 46 millimoles de -L-N-acétyl (hydroxy-5) tryptophane obtenu selon a) dans 100 ml de tétrahydrofuranne et 100 ml d'acétone. On ajoute 46 millimoles de dicyclohexyl carbodiimide et on laisse agiter 24 heures. On filtre l'urée précipitée, on évapore le solvant et on reprend à l'acétate d'éthyle qu'on lave à l'acide chlorhydrique dilué, à la soude décinormale et

enfin à l'eau. Après évaporation du solvant, le résidu est recristallisé dans le mélange éthanol/éther.

On obtient le dérivé du titre sous la forme de cristaux beiges de pF 176°C $|\alpha|_D$ = -15,40° (C=1, acétone) et $|\alpha|_D$ = -9,2° (C=1, éthanol) et dont les caractéristiques physico-chimiques sont énumérées ci-après: spectrométrie RMN (acétone $d_6$): 9,5 ppm (m) (1 H) OH phénol; 8,3 (s) (1 H) OH phénol; 7,8 à 6,7 ppm (m) (11 H) arom et -N__-; 4,7 (t) (2 H) CH (NH)CO; 3,6 ppm (s) (3 H)-$OCH_3$; 3 ppm (m) (4 H) -$CH_2$-; 1,85 ppm (s) (3 H) $CH_3CO$; spectrométrie infra-rouge (KBr): 3400-3300$^{cm-1}$ (FF) OH, NH, 1735$^{cm-1}$ (F) ester; 1650 - 1630 et 1560 -1530$^{cm-1}$ CONH (F).

**Exemple 7 - N-acétyl hydroxy-5-L-tryptophyl hydroxy-5-L-tryptophane, ester méthylique**

On dissout 22 g (94 millimoles) de -L-hydroxy-5-tryptophane ester méthylique obtenu selon H.TAMIR (J. of Neurochem. 32, 593-8 (1979)) et 24 g de -L-N-acétyl hydroxy-5-L-tryptophane obtenu selon l'exemple 6 a) dans 500 ml de tétrahydrofuranne et 50 ml de DMF, on ajoute ensuite à température ordinaire 21 g de dicyclohexylcarbodiimide en solution dans 100 ml de THF. Après 20 heures d'agitation à température ordinaire, on filtre l'urée précipitée, on évapore le solvant sous pression et on reprend dans l'éthanol d'où l'on précipite le produit par addition d'éther de pétrole. Le produit brut est chromatographié sur silice dans l'acétone. On obtient 60 % d'un produit amorphe donnant un seul spot de Rf 0,25 en CCM sur Kieselgel dans le système toluène 10, formiate d'éthyle 10, acide formique 1, éthanol 0,5.

$|\alpha|_D$ = +10,1° (C=1, acétone). Le spectre de massé et l'analyse élémentaire confirment la structure du produit qui présente d'autre part les caractéristiques suivantes:

En RMN (DMSO $d_6$) 10 ppm (m) (2 H) OH phénol; 9/8,5 ppm (m) (2 H) NH; 7,2 à 6,5 ppm (m) (8 H) arom; 4,5 ppm (m) (2 H) CH (NH) CO; 3,5 ppm (s) (3 H) $COOCH_3$; 3,0 ppm (m) (4) -$CH_2$-; 1,7 ppm (s) (3 H) $CH_3CO$.

**Exemple 8 - Hydroxy-5-L-tryptophyl-L-alanine, hydrate**

On ajoute 0,1 mole du paratoluène sulfonate de l'ester benzylique de la -L-alanine préparé selon GIBIAN (Ann. 642, 145 (1961)) à 500 ml de chloroforme pur contenant 0,1 mole de TEA.

Puis après 30 mn, on ajoute 0,1 mole de -L-N-benzyloxy carbonyl-hydroxy-5 tryptophane en solution dans 200 ml d'acétone puis 0,1 mole de dicyclohexylcarbodiimide et on agite à température ordinaire jusqu'à fin de réaction. On filtre, on lave abondamment à l'acétone et on évapore la solution organique. Le produit concrétisé dans l'éther, se présente sous forme de beaux cristaux blancs de pF = 183°C et $|\alpha|_D^{30}$ = -22,3° (C=1, acétone).

On hydrogène ce produit en solution à 10 % dans l'acide acétique en présence de palladium. Après évaporation du solvant, on recristallise dans l'eau. On obtient le dérivé du titre avec un rendement de 64 %. Le pF est de 260/300°C (dec). $|\alpha|_D^{30}$ = +11,3° (C=1, HCl N/1): la CCM dans le système B présente un seul spot de Rf = 0,45. Le dosage de l'eau par K.FISHER indique une teneur de 5,6 % confirmant l'existence du monohydrate.

**Exemple 9 - Hydroxy-5-L-tryptophane-L-valine**

A 0,1 mole de paratoluène sulfonate de l'ester benzylique de -L-valine préparé selon ZERVAS (J.O.C. 22, 1520 (1957)) mise en suspension dans 500 ml de chloroforme pur, on ajoute 0,1 mole de triéthylamine à +5°C, puis 0,1 mole de -L-N benzyloxycarbonyl- hydroxy-5 tryptophane en solution dans 200 ml d'acétone. On coule alors lentement 0,1 mole de dicyclohexylcarbodiimide en solution dans 50 ml de chloroforme et on laisse agiter à température ordinaire jusqu'à fin de condensation. Après filtration, on évapore la solution organique, puis on reprend par de l'acétate d'éthyle, qui est lavé soigneusement à l'eau acide à la soude diluée puis à l'eau. Après séchage et évaporation du solvant, le résidu est repris plusieurs fois et finalement recristallisé dans le mélange benzène-acétone.

On obtient ainsi l'ester benzylique de la -L-N-benzyl-oxycarbonyl-hydroxy-5 tryptophyl-L-valine sous forme de cristaux blancs de pF 174°C et $|\alpha|_d^{25}$ = -23,3° (C=1, acétone).

On soumet ce produit à l'hydrogénation catalytique en solution acétique en présence de palladium sur charbon. Après filtration et évaporation, le résidu repris au toluène puis au chloroforme, est recristallisé dans un mélange isopropanol-acétone. On obtient le dérivé du titre sous forme de cristaux de pF 208°C présentant un seul spot de Rf = 0,50 en CCM dans le système B. Le produit cristallise sous forme de solvate avec l'isopropanol comme le démontrent l'analyse élémentaire et le spectre RMN (DMSO) qui présente les signaux caractéristiques suivants: 10,1 ppm (s) (1 H), phénol; 8,0 ppm (m, large) (1 H)-NH-; 7,2 à 6,7 ppm (m) (4 H) arom; 5,2 ppm bande large des OH masquant CH ($NH_2$)-CO; 1,4 ppm (d) (6 H) $(CH_3)_2$ CHOH; 0,9 ppm (d) (6 H) $(CH_3)_2$-CH-.

**Exemple 10 - Hydroxy-5-L-tryptophyl-L-leucine**

A 500 ml de chloroforme, on ajoute 0,1 mole (39,4 g) de paratoluène sulfonate de l'ester benzylique de la -L-leucine préparé selon ZERVAS (J.O.C. 22, 1520 (1957)) et 0,1 mole de triéthylamine. Après une heure d'agitation, on coule 0,1 mole (35,4 g) de N-benzyloxycarbonyl-L-hydroxy-5-tryptophane dissous dans 200 ml d'acétone et 0,1 mole de dicyclohexylcarbodiimide en solution dans 50 ml de chloroforme et on maintient la température ordinaire jusqu'à fin de réaction. Après filtration, évaporation, reprise à l'acétate d'éthyle et lavages habituels de cette solution organique, on évapore à sec. Le résidu amorphe est d'abord repris à l'éther, puis recristallisé dans l'isopropanol.

On obtient ainsi l'ester benzylique de la -L-N-benzyloxycarbonyl-hydroxy-5-tryptophyl-L-valine sous forme de cristaux de pF = 159°C. $|\alpha|_D^{25}$ = -15,2° (C=1, acétone) qui est soumis à l'hydrogénolyse catalytique en solution acétique en présence de palladium. Le résidu d'évaporation est finalement recristallisé dans l'éthanol. On obtient le dérivé du titre sous forme de solvate contenant une molécule d'éthanol, de pF = 164°C donnant en CCM un spot de Rf = 0,45 dans le système B.

Le spectre de RMN ($D_2O$) présente les signaux caractéristiques suivants: 7,4 ppm (d) (1 H) (J = 9 Hz); 7,3 ppm (s) (1 H); 7,15 ppm (d) (1 H) J' = 2 Hz; 6,85 ppm (dd) (1 H), J' = 2 Hz J = 9 Hz, (arom); 4,3 ppm (m) (2 H) -CH(NH)-CO; 3,7 ppm (q) (2 H) $CH_3CH_2OH$; 3,2 ppm (m) (2 H)-$CH_2$-CO; 1,5 ppm (m) (2 H)-$CH_2$-; 1,15 ppm (t) (3 H) $CH_3CH_2OH$; 0,7 ppm (m) (7 H)-$CH(CH_3)_2$-.

**Exemple 11 - Hydroxy-5-L-tryptophyl-L-proline**

On dissout 0,1 mole de chlorhydrate de l'ester benzylique de la -L-proline obtenu selon RAMACHANDRAN (Journal of Organic Chemistry 28, 173/177 (1963)) dans 500 ml de chloroforme pur et on ajoute à froid 0,1 mole de triéthylamine. Après quelques minutes, le milieu réactionnel est additionné de 0,1 mole de -L-N-benzyloxycarbonyl-hydroxy-5-tryptophane en solution dans 200 ml d'acétone, puis de 0,1 mole de dicyclohexylcarbodiimide dissous dans 50 ml de chloroforme. On laisse la température revenir à l'ambiante et on agite jusqu'à fin de réaction. On filtre, on évapore à sec et on reprend à l'acétate d'éthyle qui est lavé de façon habituelle, séché, puis évaporé. Le produit est ensuite chromatographié sur colonne de silice dans le mélange chloroforme-acétone, on obtient ainsi 40 % de -L-N-benzyloxycarbonyl-hydroxy-5-tryptophyl-L-proline ester benzylique $|\alpha|_D$ = -33,9° (C=1, acétone) que l'on soumet à l'hydrogénolyse dans l'acide acétique en présence de palladium.

Le produit final est recristallisé dans un mélange acétone éthanol. On obtient ainsi la -L-hydroxy-5-tryptophyl-L-proline de pF 162/3° présentant en CCM dans le système B un seul spot de Rf = 0,7. $|\alpha|_D$ = -8,4° (C=1, HCl N/1).

**Exemple 12 - Hydroxy-5-L-tryptophyl-L-phényl-alanine**

On ajoute 86 millimoles (36,75 g) de paratoluène sulfonate de l'ester benzylique de la -L-phényl-alanine préparé selon ZERVAS (J.O.C. 22, 1520 (1957)) à 500 ml de chloroforme puis on ajoute 86 millimoles de triéthylamine à 0/+5°C. Après quelques minutes, on ajoute toujours à froid 86 millimoles de -L-N-benzyloxycarbonyl-hydroxy-5-tryptophane en solution dans 500 ml d'acétone et 90 millimoles de dicyclohexylcarbodiimide dissous dans 50 ml de chloroforme. Après une nuit à température ambiante, on évapore, on reprend à l'acétate d'éthyle, on filtre l'urée formée et on lave la solution organique de façon habituelle. Après séchage et évaporation, on concrétise dans l'éther puis on recristallise dans l'éthanol. On obtient ainsi 55 % de -L-N-benzyloxycarbonyl hydroxy-5-tryptophyl-L-phényl-alanine ester benzylique cristallisé de pF = 202°. Ce dérivé est hydrogéné en solution à 10 % dans l'acide acétique en présence de palladium puis recristallisé dans l'éthanol. On obtient ainsi 80 % de -L-hydroxy-5-tryptophyl-L-phényl alanine de pF = 260°C (dec) présentant un seul spot en CCM dans le système B de Rf = 0,4 un $|\alpha|_D^{25}$ = -31° (C=1, DMF). L'analyse élémentaire confirmée par la RMN démontre l'existence d'un solvate contenant une molécule d'éthanol.

**Exemple 13 - Hydroxy-5-L-tryptophyl hydroxy-5-L-tryptophane**

a) Ester benzylique du -L-hydroxy-5-tryptophane

On ajoute 0,2 M de -L-hydroxy-5-tryptophane à 500 ml d'alcool benzylique puis on ajoute lentement sous vive agitation 75 ml d'acide phosphorique concentré. On chauffe alors le milieu réactionnel pendant 6 heures à 80/90°C, puis après refroidissement on coule dans l'eau glacée légèrement chlorhydrique. On extrait l'alcool benzylique en excès par de l'éther puis on neutralise la solution aqueuse. Le produit est extrait à l'éther puis après évaporation, est purifié par chromatographie sur colonne de silice dans l'acétate d'éthyle. On obtient 30

% d'une huile jaune pure.

b) N-benzyloxycarbonyl hydroxy-5-L-tryptophyl-hydroxy-5-L-tryptophane, ester benzylique

On ajoute 40 millimoles du produit précédemment obtenu selon a) à 40 millimoles de -L-N-benzyloxycarbonyl hydroxy-5-tryptophane dissous dans 500 ml de chlorofrome, puis on coule lentement à +5°C 40 millimoles de dicyclohexylcarbodiimide dissous dans 50 ml de THF. On agite à température ambiante jusqu'à fin de réaction, on filtre, on évapore et on cristallise dans le chloroforme. On obtient ainsi le dérivé du titre à l'état pur . pF = 187/8°C.

c) Hydroxy-5-L-tryptophyl-hydroxy-5-L-tryptophane, hemi hydrate

On hydrogène le produit précédent dans l'alcool isopropylique à 60°C sous 30 bars en présence de palladium à 5 % sur charbon. Après filtration et évaporation, on recristallise dans l'eau. On obtient ainsi le dérivé du titre à l'état d'hémi hydrate de pF = 360° (dec): $|\alpha|_D^{25}$ = -42,2° (C=1, éthanol) présentant en CCM un seul spot de Rf = 0,80 dans le système B et Rf = 0,1 dans le système toluène 10, formiate d'éthyle 10, acide formique 1, éthanol 1. Le spectre de RMN en solution dans DMSO d6 présente les signaux caractéristiques suivants: 11 ppm (m) (2 H) OH phénoliques: 8,5 ppm (m) (4 H) échangeables. 7,7 à 6,7 ppm (m) (10 H) (arom); 4,5 ppm (m) (1 H) -CH (NH)-CO; 3,5 ppm (m) (1 H) CH ($CH_2$) CO; 3,00 ppm (m) (4 H) -$CH_2$-.

**Exemple 14 - Hydroxy-5-L-tryptophyl-L-tryptophane**

a) -N benzyloxycarbonyl hydroxy-5-L-tryptophyl-L-tryptophane, ester benzylique

On ajoute 18,3 g (51,7 mM) de -L-N-benzyloxycarbonyl hydroxy-5-tryptophane dissous dans 100 ml d'acétone pure à 15,2 g d'ester benzylique du -L-tryptophane synthétisé selon JACS 76, 5781 (1954) en solution dans 285 ml de chloroforme pur puis après addition de 10,8 g de dicyclohexylcarbodiimide, on laisse sous agitation pendant 48 heures à température ambiante. Après filtration et évaporation du solvant, on cristallise dans le chloroforme, puis on recristallise dans l'alcool isopropylique.

Le produit pur présente un seul spot en CCM dans le système toluène 10, formiate d'éthyle 10, HCOOH 1 de Rf = 0,5.

b) Hydroxy-5-L-tryptophyl-L-tryptophane

On soumet 8,5 g du produit précédemment décrit à l'hydrogénolyse en suspension dans 200 ml d'acide acétique en présence de palladium à 5 % sur charbon. Après filtration et évaporation de la solution résultante, on reprend le résidu au benzène puis à l'éther. On obtient 5,0 g de cristaux légèrement gris présentant un seul spot en CMM dans le système A de Rf = 0,50.

**Exemple 15 - -L-hydroxy-5-tryptophyl-O-tert-butyl-L-sérine-, ter-butylester**

a) N-benzyloxycarbonyl-O-ter-butyl-L-sérine-tert-butylester

On met en suspension 24 g (0,1 M) de N-benzyloxycarbonyl -L-sérine préparé selon Houben Weyl XV 1 (49), dans 220 ml de chlorure de méthylène contenant 1,5 ml d'acide sulfurique pur et 80 g d'isobutylène. L'appareil est fermé de façon étanche et la solution est agitée 3 jours à température ordinaire. Après évaporation de l'excès de réactif, on lave la solution organique au bicarbonate, puis on sèche et on évapore.

b) O-tert-butyl-L-sérine-ter-butylester

Le produit précédent est soumis à hydrogénolyse dans l'éthanol en présence de palladium à 5 % sur charbon actif. Après filtration et évaporation, on distille le dérivé du titre à t = 55°C/0,25 mm avec un rendement de 55 %. Le produit pur ne présente qu'un seul spot de Rf = 0,1 dans le système toluène 10, formiate d'éthyle 1, acide formique 1.

c) N-benzyloxycarbonyl-L-tryptophyl-O-ter-L-sérine-tert-butylester

On dissout 16,5 g (75 mM) du produit précédent dans 150 ml de chloroforme pur et on ajoute à 27 g (75 mM) de N-benzyloxycarbonyl-L-hydroxy-5 tryptophane dissous dans 150 ml d'acétone, après addition de 76 mM de dicyclohexylcarbodiimide dissous dans 200 ml de chloroforme. On laisse agiter à température ordinaire jusqu'à fin de condensation. Après filtration et évaporation, on reprend par l'acétate d'éthyle qu'on lave par HCl dilué, NaOH diluée puis à l'eau. Le produit est ensuite purifié par chromatographie sur colonne de silice par élution avec un mélange chloroforme/acétone. On obtient 60 % de produit pur présentant un seul spot de Rf = 0,9 par CCM dans le système A.

d) L-hydroxy-5-tryptophyl-O-ter-butyl-L-sérine-tert-butylester

On hydrogène 6,36 g du produit précédent dans 65 ml d'acide acétique. Après filtration et évaporation, on reprend au benzène puis à l'éther. On obtient ainsi 80 % du dérivé du titre à l'état pur présentant un seul spot en CCM dans le système A de Rf = 0,60.

Le spectre de RMN enregistré dans le DMSO d6 présente les signaux caractéristiques suivants: 1,1 et 1,4 ppm 2 (s) (9 H); 3 à 3,6 ppm (m) (5 H); 2-$\underline{CH_2}$- et $\underline{CH}$ ($NH_2$)-COOH; 4,5 ppm (m) (1 H) $\underline{CH}$ (NH-)$CH_2$; 5,8 ppm (m) $NH_2$; 6,6 à 7,4 ppm (5 H) (arom); 8,3 ppm (m) 1H -$\underline{NH}$-.

$|\alpha|_D^{25}$ = -10° (C=1, DMF).

**Exemple 16 - L-hydroxy-5-tryptophyl-L-sérine**

On ajoute 3,5 g du produit précédent à 20 ml d'une solution saturée d'acide chlorhydrique dans l'acide acétique. Après une heure à température ordinaire, on coule dans 150 ml d'éther et on essore le précipité. On obtient ainsi le dérivé du titre sous forme d'un produit cristallisé blanc à gris pâle présentant un seul spot en CCM dans le système A de Rf = 0,20. Le spectre de RMN enregistré dans le DMSO d6 présente les signaux caractéristiques suivants: 7,2 et 6,5 ppm (m) (4 H) arom; 4,5 ppm (m) (2 H) -CH (NH)-CO; 3,7 ppm (m) (2 H), CH-$CH_2$-O; 3,1 ppm (m) (2 H) $CH_2OH$.

**Exemple 17 - L-hydroxy-5 tryptophyl-L-sérine par l'ester cyanométhylique du sel de dane du L-hydroxy-5 tryptophane**

a) L-N (méthyl-1 acétyl-2 vinyl) hydroxy-5 tryptophane ester cyanométhylique

On dissout 17,6 g (80 mM) de -L-hydroxy-5 tryptophane dans 80 ml de diméthyl formamide, on ajoute 80 mMoles de tétraméthylguanidine et on agite 30 mn à température ordinaire.

On coule ensuite dans la solution réactionnelle 8,0 g (80 mMoles) d'acétyl-acétone puis après 2 heures d'agitation à température ordinaire, 6,1 g (80 mM) de chloroacétonitrile.

On agite à température ordinaire jusqu'à fin de réaction, soit environ 48 heures. On coule ensuite le milieu réactionnel dans une solution aqueuse de bicarbonate de sodium, qu'on extrait par l'acétate d'éthyle. Après lavage de la solution organique par une solution diluée de bicarbonate de sodium puis par de l'eau, on sèche sur sulfate de soude et on évapore à sec. On obtient ainsi 22,9 g (84 %) du dérivé du titre présentant un seul spot en CCM de Rf = 0,9 dans le système A et de Rf = 0,4 dans le système chloroforme 5, acétate d'éthyle 20. Le spectre de RMN enregistré dans $CD_3CN$ présente les signaux caractéristiques suivants: 6,6 à 7,3 ppm (m) (5 H) (arom); 5,0 ppm (s) (1 H) = CH-CO; 4,8 ppm (s) (2 H) $OCH_2$ CN; 4,6 ppm (m) (1 H) $CH_2$CH (NH) COO; 3,2 ppm (m) (2 H) $CH_2$-CH(NH)COO; 2 ppm (2s) (6 H) $CH_3$-C=, $CH_3$ C=O.

b) N-(méthyl-1-acétyl-2 vinyl)-hydroxy-5-L-tryptophyl-O-tert-butyl-L-sérine-O-tert-butylester

On ajoute 13,68 g (63 mM) de O-tert-butyl-L-sérine-O-tert-butylester préparé selon l'exemple 15 b) et 21,48 g (63 mM) de l'ester réactif du L-5-hydroxy-tryptophane préparé précédemment selon a) à un mélange de 60 ml d'acétonitrile et 100 ml d'acétate d'éthyle auquel on a ajouté 0,1 ml d'acide acétique glacial. On agite ensuite à température ordinaire jusqu'à fin de condensation. Après filtration d'un léger insoluble, on évapore à sec et et on purifie par chromatographie sur colonne de silice en éluant par un mélange de chloroforme/éthanol.

On isole ainsi le dérivé du titre à l'état pur avec un rendement proche de 40 %. La chromatographie sur couche mince de silice dans le système toluène 10, formiate d'éthyle 10, acide formique 1, donne un seul sport de Rf = 0,40.

c) Hydroxy-5-L-tryptophyl-L-sérine

On dissout 3,4 g (6,8 mM) du dérivé précédemment obtenu selon b) dans 25 ml d'acide acétique saturé d'acide chlorhydrique et on agite la solution pendant quelques heures à température ordinaire, puis par précipitation dans un excès d'éther éthylique, filtration et séchage du précipité, on obtient le dérivé du titre à l'état pur, identique au produit obtenu selon l'exemple 16.

**Exemple 18 - Hydroxy-5-L-tryptophyl-L-arginine**

a) N-benzyloxycarbonyl-hydroxy-5-L-tryptophyl-ω-nitro-L-arginine ester benzylique

A une solution de 24,15 g (68,2 mM) de N-benzyloxy-carbonyl-L-hydroxy-5-tryptophane dans 135 ml d'acétone, on ajoute 22,7 g (70 mM) d'ω-nitro-L-arginine ester benzylique dissous dans 170 ml de chloroforme.

On coule ensuite 70 mMoles de dicyclohexylcarbodiimide en solution dans le chloroforme et on agite à température ordinaire jusqu'à fin de réaction. Après filtration de la dicyclohexylurée et évaporation du solvant, on reprend par l'acétate d'éthyle qu'on lave ensuite par l'acide chlorhydrique normal, par la soude, puis à l'eau. Après séchage, évaporation et reprise à l'éther, on obtient le dérivé du titre à l'état pur présentant en CCM un seul spot de Rf = 0,6 dans l'acétonitrile. La spectrométrie de RMN dans $CD_3CN$ présente les signaux caractéristiques suivants: 7,2 ppm (s) (10 H) benzyl; 7,4 à 6,6 ppm (m) (5 H) (arom); 5,0 ppm (2 x s) (4 H) 2 x -$CH_2$ benzyl; 4,5 ppm (m) (2 H) 2-CH (NH) CO; 3,2 ppm (m) (4 H).

b) Hydroxy-5-L-tryptophyl-L-arginine

On soumet à l'hydrogénolyse 3,7 g (5,7 mM) du dérivé obtenu selon a) en solution dans l'acide acétique en présence de palladium à 5 % sur charbon. Après filtration et évaporation du solvant, on reprend le produit dans un mélange eau/éthanol, on obtient alors le dérivé du titre à l'état pur. La CCM de ce dérivé donne un seul spot de Rf = 0,05 dans le système toluène 10, formiate d'éthyle 10, acide formique 1. Le spectre de RMN enregistré dans DMSO d6 présente les signaux caractéristiques suivants: 7,5 à 6,5 ppm (m) (4 H) (arom); 4,4 ppm (m) (2 H) -CH-(NH)CO; 3,2 ppm (m) (4 H) $CH_2$-CH(NH)CO et N-$CH_2$-; 1,7 ppm (m) (4 H) -$CH_2$-$CH_2$-.

**Exemple 19 - Hydroxy-5-L-tryptophyl-glycine**

On dissout 0,1 mole (33,7 g) de paratoluène sulfonate du glycinate de benzyle (préparé selon ZERVAS, Journal of Organic Chemistry 22, 1520 (1957)) dans 500 ml de chloroforme pur contenant 0,1 mole de triéthylamine. Après 30 mn, on ajoute 34 g (0,1 mole) de -L-N-benzyloxycarbonyl hydroxy-5-tryptophane, dissous dans 200 ml d'acétone puis on ajoute à +5°C 0,1 mole de dicyclohexylcarbodiimide, dissous dans 50 ml de chloroforme. On agite à température ordinaire jusqu'à fin de réaction (environ 24 heures). On filtre ensuite, on évapore, on reprend par de la méthyléthylcétone, on lave à l'eau acide, à la soude diluée et enfin à l'eau. Après séchage et évaporation, on cristallise dans un mélange chloroforme-ethanol. On obtient un produit pur en CCM de pF = 114°C et $|\alpha|_D^{25}$ = -31,2 (C=1, acétone) qui est hydrogéné en solution dans l'acide acétique en présence de palladium. En fin d'hydrogénation, on filtre, on évapore, on réempâte plusieurs fois dans l'acétone puis on cristallise dans l'éthanol. On obtient le dérivé du titre à l'état de solvate contenant une molécule d'éthanol de pF 178° présentant en CCM une seule tache de Rf = 0,15 dans le système B.

$|\alpha|_D^{25}$ = 27,7° (C=1, DMF). Spectre RMN enregistré dans $D_2O$: 7,5 à 6,8 ppm (m) (4 H) (arom). 4,8 ppm bande large H mobiles; 4,2 ppm (m) (1 H) -<u>CH</u> CONH; 3,7 ppm (q) (2 H), $CH_3\underline{CH_2}$ OH; 3,7 ppm (d) (2 H) $-CH_2-CH-$; 3,2 ppm (d) (2 H) $NH\underline{CH_2}CO-$; 1,1 ppm (t) (3 H) $\underline{CH_3}-CH_2-OH$;

**Exemple 20 - Acide hydroxy-5-L-tryptophyl-Y-amino butyrique**

a) <u>N-benzyloxycarbonyl-hydroxy-5-L-tryptophyl-γ-amino butyrate de tert-butyle</u>

On met en solution 85 millimoles de N-benzyloxycarbonyl-hydroxy-5-L-tryptophane et 85 mM de γ-amino butyrate de tert-butyle dans 170 ml d'acétone et 300 ml de chloroforme pur. On ajoute ensuite 17,7 g (86 mM) de dicyclohexylcarbodiimide en solution dans 80 ml de chloroforme et on agite à température ordinaire jusqu'à fin de condensation. Après évaporation, reprise à l'acétate d'éthyle et traitement habituel, on obtient 40 g de produit brut qu'on purifie par chromatophraphie sur colonne de silice en éluant avec un mélange dichlorométhane/acétone. On obtient ainsi 48 % de produit pur présentant un seul spot par CCM sur Kieselgel dans le système chloroforme 20, acétone 6 de Rf = 0,45. Le spectre de RMN est enregistré en solution dans $CDCl_3$: 8,4 ppm (s) (1 H) - OH; 7,3 ppm (s) (5 H) benzyl; 7,2 à 6,6 ppm (m) (4 H) arom; 6,4 ppm (m) (1 H) et 5,9 ppm (d) (1 H) - NH; 5 ppm (s) (2 H) $CH_2$-Ph; 4,4 ppm (m) (1 H) CH (NH)-CO; 3,1 ppm (m) (4 H) $\underline{CH_2}$-CH (-NH) CO et NH $\underline{CH_2}$; 2,1 à 1,8 ppm (m) 4 H: $\underline{CH_2}-\underline{CH_2}$; 1,4 ppm (s) (9 H) tert.-but.

b) <u>Hydroxy-5-L-tryptophyl-γ-amino-butyrate de tert-butyl</u>

On hydrogène le produit précédemment obtenu dans 120 ml d'acide acétique en présence de 1,2 g de palladium à 5 %/C. Après filtration du catalyseur, on évapore à sec et on reprend à l'éther. On obtient ainsi le dérivé du titre à l'état pur avec un rendement quantitatif.

Sa CCM sur Kieselgel dans le système A ne révèle qu'un seul spot de Rf = 0,5. Son spectre de RMN enregistré dans DMSO d6 présente les signaux caractéristiques suivants: 10,5 ppm (s) (1 H) NH indol; 8 ppm (1 H) (m)-OH; 7,2 à 6,5 ppm (m) (4 H) arom; 3,5 ppm (1 H) (m) <u>CH</u> (NH-) CO; 3 ppm (m) (2 H) $\underline{CH_2}$-CH (NH)-; 2,9 ppm (m) (2 H) NH-$\underline{CH_2}$; 2,2 à 1,3 ppm (m) 6 H; 1,4 ppm (s) (9 H)- -O-t-But.

c) <u>Acide hydroxy-5-L-tryptophyl-γ-amino butyrique</u>

On dissout le produit précédemment obtenu dans 8 ml d'acide acétique saturé en acide chlorhydrique à environ 10°C, on agite deux heures à température ordinaire puis on évapore à sec. Le produit pur est purifié par chromatographie sur gel Sephadex G 10 dans l'acide formique N/10. On obtient ainsi le dérivé du titre à l'état pur. Sa chromatographie sur plaque Kieselgel ne présente qu'un seul spot de Rf = 0,3 dans le système A. Spectre de RMN dans DMSO d6: 8,4 ppm (s) (2 H) -NH; 7,2 à 6,5 ppm (m) (4 H) arom; 3,8 ppm (m) (1 H) <u>CH</u> (NH)-CO; 3,1 ppm (m) (4 H) $\underline{CH_2}$ CH(NH) et NH $\underline{CH_2}$; 2,2 ppm (dd) (2 H) $-CH_2-$; 1,7 ppm (m) (2 H) $-CH_2-$.

**Exemple 21 - Hydroxy-5-L-tryptophanamide, hydrate**

On dissout 0,115 mole (69,3 g) de -L-N-benzyloxy-carbonyl-hydroxy-5 tryptophane ester pentachlorophénylique, dans 300 ml d'éthanol anhydre contenant 20 % d'ammoniac. Après une nuit à +5°C, on filtre le phénol, on lave à l'éther et on évapore la solution.

On réempâte dans l'éther isopropylique et on filtre le Z-Trp (5 HO) $NH_2$ pur de pF = 171°C avec un rendement presque quantitatif. Ce produit est hydrogéné dans l'alcool isopropylique en présence de palladium sur charbon. Après filtration et évaporation, le produit est repris dans l'eau, chromatographié sur gel, puis lyophilisé. La CCM dans A présente un seul spot à Rf = 0,45. Le spectre de RMN enregistre dans DMSO d6 présente les signaux suivants: 10,5 ppm (s) large (1 H), OH phénol; 7,4 à 6,4 ppm (m) (7 H), 5 arom et 2 H mobiles; 4,3 ppm bande large, $H_2O$ et $NH_2$; 3,4 ppm (m) (1 H) <u>CH</u>-CO; 2,8 ppm (m) (2 H) $-CH_2-$; le spectre intrarouge (KBr) comporte une forte absorption de 3500 à 2500 (fortes liaisons hydrogènes) et une bande caractéristique à 1670$^{cm-1}$. $|\alpha|_D^{25}$ -8,47° (C=1, éthanol).

**Exemple 22 - Hydroxy-5-L-tryptophyl-L-histidine, ester méthylique**

A 50 millimoles d'ester méthylique de la -L-histidine préparé selon N.C.DAVIS (J.Biol.Chem. 223, 935 (1956)) en solution dans 200 ml d'acétonitrile, on ajoute 50 millimoles de N-benzyloxycarbonyl-hydroxy-5-L-tryptophane en solution dans 150 ml d'acétone. On traite à 0°C par 52 millimoles de DCC en solution dans 100 ml de chloroforme. On maintient à température ambiante jusqu'à fin de réaction. Après évaporation, reprise à l'acétate d'éthyle et traitement habituel, on obtient 80 % de produit brut que l'on purifie par chromatographie sur silice en éluant par le mélange dichlorométhane/acétone.

Le produit purifié est soumis à l'hydrogénolyse en présence de palladium/charbon en solution dans le méthanol. Après filtration et évaporation du solvant, on purifie le produit par chromatographie sur gel G 10 en éluant à l'acide formique M/1. On obtient ainsi le dérivé du titre à l'état de formiate. La CCM du produit présente un seul spot de Rf 0,3 dans le système A. En RMN, on note les signaux caractéristiques suivants (DMSO d6): 9,7 ppm (m) (1 H) NH indol; 8,7 ppm (s) (1 H) NH imidazol; 7,5 et 6,5 ppm (m) (4 H) indol; 7,5 (s) (1 H) imidazol; 4,5 ppm (m) (1 H) CH-(NH)-CO; 4,2 ppm (m) (1 H) CH $(NH_2)$-CO; 4,0 ppm (s) (3 H) $OCH_3$; 3,5 ppm (m) (4 H) $-CH_2-$.

Ces nouveaux produits conformes à la présente invention manifestent des activités anxiolytiques, analgésiques, antihypertensives et antiépileptiques, se révélant ainsi être de remarquables médicaments utilisables en thérapeutique humaine ou vétérinaire.

Le compte-rendu pharmacologique qui suit met en évidence l'activité de ces composés sur le système nerveux central.

Le dosage biochimique des teneurs cérébrales en -L-hydroxy-5-tryptophane (L-5 HTP), en sérotonine (5-HT) et en son premier métabolite l'acide hydroxy-5 indol acétique (5-HIAA) a été réalisé une heure après administration à des rats adultes d'une dose unique selon la technique de CURZON et GREEN. Trois doses ont été étudiées, 10,25,50 mg/kg en utilisant 10 animaux par dose. Le Tableau I ci-après indique les pourcentages d'augmentation du 5-HTp, 5-HT, 5-HIAA par rapport aux teneurs trouvées chez les animaux témoins:

**Tableau I**

| | % par rapport aux témoins en: | | | | | | | | |
|---|---|---|---|---|---|---|---|---|---|
| | -L-5 HTP aux doses de | | | 5-HT aux doses de | | | 5-HIAA aux doses de | | |
| EXEMPLE | 10 mg/kg | 25 mg/kg | 50 mg/kg | 10 mg/kg | 25 mg/kg | 50 mg/kg | 10 mg/kg | 25 mg/kg | 50 mg/kg |
| 2 | 200 | 600 | 1000 | 5 | 5 | 10 | | | |
| 3 | 100 | 400 | 1100 | 10 | | 200 | 50 | 150 | 200 |
| 4 | 200 | 550 | 2500 | 5 | 10 | 60 | | | |
| 5 | 200 | 450 | 1200 | 12 | 10 | 40 | 55 | 160 | 170 |
| 6 | | | | | 5 | 5 | | | |
| 7 | 10 | | 30 | | | | 10 | 20 | 20 |
| 8 | 200 | 3000 | 2400 | | 5 | | 75 | 140 | 400 |
| 9 | 300 | 900 | 1700 | 12 | 40 | 45 | 70 | 250 | 320 |
| 10 | 200 | 600 | 2000 | | 12 | 45 | 55 | 100 | 350 |
| 11 | 200 | 700 | 1600 | 10 | | 20 | | 150 | 300 |
| 12 | 170 | 500 | 1100 | | | 25 | | 140 | 180 |
| 13 | | 50 | 50 | | 5 | 5 | | | 10 |
| 14 | 50 | 100 | 200 | | 12 | 10 | | | |
| 15 | 200 | 400 | 900 | | | | | 60 | 150 |
| 16 | 170 | | 900 | | | 15 | 10 | 30 | 150 |
| 18 | 100 | 300 | 500 | 12 | 30 | 30 | 10 | 15 | 15 |
| 19 | | 700 | 1300 | 30 | 12 | 30 | 75 | 170 | 350 |
| 20 | 60 | 200 | 400 | 5 | 5 | 15 | 40 | 100 | 150 |
| 21 | 125 | 250 | 700 | | | 20 | 30 | 50 | 170 |
| 22 | | 200 | 300 | | 15 | 15 | 40 | 140 | 300 |

In vivo, tous les composés selon l'invention se sont révélés analgésiques dans le test à la plaque chauffante selon LESPAGNOL A. et MERCIER J. (Ann.Phar.Fr (1950) 8, 241-251) à des doses comprises entre $1,5\text{-}10^{-3}$ et $1.10^{-6}$ Mole/kg, l'effet analgésique se prolongeant au-delà de deux heures après administration.

D'autre part, les dérivés conformes à l'invention présentent chez la souris une toxicité aiguë très inférieure à celle du L-5-HTP. Ainsi, les $DL_{50}$ de ces composés administrés per os à la souris sont supérieures à 1500 mg/kg pour les plus toxiques et atteignent même 3000 mg/kg.

Il résulte de la présente description que les dérivés selon l'invention offrent des activités pharmacologiques et cliniques particulièrement intéressantes dans toutes les affections où le système sérotoninergique est déficient et par là ils constituent des médicaments permettant de guérir les maladies résultant de ce dérèglement, compte tenu de leur activité élevée et de leur faible toxicité.

**Revendications**

1. Dipeptides de -L-5-hydroxy-tryptophane de formule générale I ci-après:

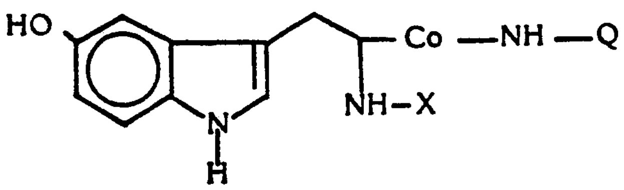

(I)

dans laquelle:
X représente l'hydrogène ou un radical acyle inférieur,
Q représente un reste d'amino-acide libre, à l'exception de tyrosine, ou estérifié, y compris la tyrosine,
ainsi que leurs sels d'addition acides ou basiques pharmaceutiquement compatibles.

2. Dipeptides selon la Revendication 1, dans laquelle Q représente un reste d'acide aminé naturel et X représente l'hydrogène ou un radical acyle inférieur.

3. Dipeptides selon la Revendication 1, dans laquelle Q représente un reste d'acide aminé non naturel et X représente l'hydrogène ou un radical acyle inférieur.

4. Dipeptides selon l'une quelconque des Revendications 1 à 3, dans lesquelles Q représente un reste d'acide aminé dicarboxylique.

5. Dipeptides selon l'une quelconque des revendications 1 à 3, dans lesquelles Q représente un reste d'acide aminé basique.

6. Dipeptides selon l'une quelconque des Revendications 1 à 3, dans lesquelles Q représente un reste d'aminoacide alcool.

7. Dipeptides selon les Revendications 4, 5, 6 dans lesquelles les fonctions réactives sont substituées.

8. Procédé de préparation des dipeptides selon les Revendications 1 à 7, caractérisé en ce que:
a) l'on prépare un dérivé bloqué sur l'azote en α du L-5-hydroxy-tryptophane,
b) l'on prépare un dérivé bloqué d'un acide aminé en ne laissant subsister que le groupe $-NH_2$ comme fonction réactive,
c) l'on condense les deux produits bloqués en présence d'un agent de condensation dans un solvant approprié à une température comprise entre 0 et 30° C,
d) l'on déploque éventuellement les fonctions protégées.

9. Procédé selon la Revendication 8, caractérisé en ce que les groupes du blocage sur l'azote en α du -L-5-hydroxytryptophane (HTP) sont choisis parmi les radicaux carbobenzyloxy-, para-méthoxy-benzyloxy-carbonyle, terbutyloxycarbonyle, les sels de DANE, avec l'acétylacétone ou l'acétoacétate d'éthyle.

10. Procédé selon la Revendication 8, caractérisé en ce que le blocage de la ou des deux fonctions acide de l'acide aminé est effectué à l'aide de composés choisis dans le groupe qui comprend les esters d'alcools primaires, secondaires ou tertiaires, les esters benzyliques.

11. Procédé selon la Revendication 8, caractérisé en ce que le -L-5-hydroxy-tryptophane bloque sur l'azote en α est utilisé sous forme d'un ester réactif, ledit ester étant choisi parmi les esters cyanométhyliques, 4-nitrophényliques, pentachlorophényliques.

12. Procédé selon la Revendication 8, caractérisé en ce que la condensation des produits bloqués est effectuée en présence du dicyclohexylcarbodiimide dans un solvant pris dans le groupe qui comprend le diméthylformamide, le diméthylsulfoxyde, la pyridine, le chloroforme, le dichlorométhane, le tétrahydrofuranne et l'acétone.

13. Procédé selon la Revendication 8, caractérisé en ce que le déblocage des fonctions protégées est effectué par hydrogénation catalytique ou hydrolyse ménagée.

14. Médicaments à usage humain ou vétérinaire, caractérisés en ce qu'ils contiennent ou sont constitués d'un ou plusieurs dérivés répondant à la formule générale I ci-après:

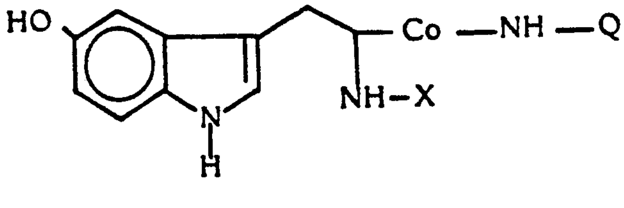

( I )

dans laquelle:
X représente l'hydrogène ou un radical acyle inférieur,
Q représente un reste d'amino-acide libre ou estérifié, ainsi que leurs sels d'addition acides ou basiques pharmaceutiquement compatibles.

**Patentansprüche**

1. Dipeptide des L-5-Hydroxytryptophans der nachstehenden allgemeinen Formel I:

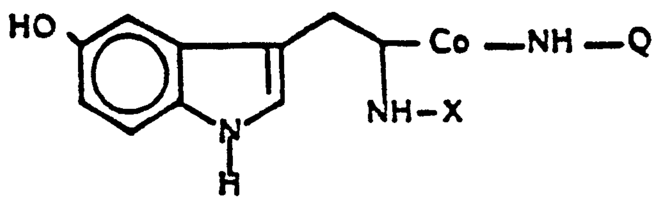

(I)

in der
X Wasserstoff oder ein niederes Acylradikal und
Q den Rest einer freien Aminosäure mit Ausnahme von Tyrosin oder einer veresterten Aminosäure, einschließlich Tyrosin,
bedeuten,
sowie deren Additionssalze mit pharmazeutisch annehmbaren Säuren oder Basen.

2. Dipeptide gemäß Anspruch 1, in denen Q den Rest einer natürlich vorkommenden Aminosäure und X Wasserstoff oder ein niederes Acylradikal bedeuten.

3. Dipeptide gemäß Anspruch 1, in denen Q den Rest einer nicht in der Natur vorkommenden Aminosäure und X Wasserstoff oder ein niederes Acylradikal bedeuten.

4. Dipeptide gemäß einem der Ansprüche 1 bis 3, in denen Q den Rest einer Aminodicarbonsäure bedeutet.

5. Dipeptide gemäß einem der Ansprüche 1 bis 3, in denen Q den Rest einer basischen Aminosäure bedeutet.

6. Dipeptide gemäß einem der Ansprüche 1 bis 3, in denen Q den Rest eines Aminosäurealkohols bedeutet.

7. Dipeptide gemäß den Ansprüchen 4, 5 und 6, in denen die reaktiven funktionellen Gruppen substituiert sind.

8. Verfahren zur Herstellung der Dipeptide gemäß den Ansprüchen 1 bis 7, dadurch gekennzeichnet, daß man
a) ein Derivat des L-5-Hydroxytryptophans herstellt, in dem der α-Stickstoff blockiert ist,
b) ein blockiertes Derivat einer Aminosäure herstellt, in dem man nur die Aminogruppe als reaktive Gruppe beläßt,
c) die beiden blockierten Produkte in Gegenwart eines Kondensationsmittels in einem geeigneten Lösungsmittel bei einer Temperatur zwischen 0 und 30° C kondensiert,
d) gegebenenfalls die geschützten funktionellen Gruppen entblockiert.

9. Verfahren gemäß Anspruch 8, dadurch gekennzeichnet, daß die Blockierungsgruppen für den Stickstoff des L-5-Hydroxytryptophans (HTP) aus der Gruppe Carbobenzyloxy, p-Methoxybenzyloxycarbonyl, tert.-Butyloxycarbonyl, den DANE-Salzen mit Acetylaceton oder Acetessigsäureethylester ausgewählt werden.

10. Verfahren gemäß Anspruch 8, dadurch gekennzeichnet, daß die Blockierung der Säurefunktion oder der beiden Säurefunktionen der Aminosäure unter Zuhilfenahme von Verbindungen, ausgewählt aus der Gruppe, bestehend aus Estern primärer, sekundärer oder tertiärer Alkohole, Benzylestern, bewirkt wird.

11. Verfahren gemäß Anspruch 8, dadurch gekennzeichnet, daß das an dem α-Stickstoff blockierte L-5-Hydroxytryptophan in Form eines reaktionsfähigen Esters eingesetzt wird, der aus der Gruppe Cyanmethylester, 4-Nitrophenylester und Pentachlorphenylester ausgewählt worden ist.

12. Verfahren gemäß Anspruch 8, dadurch gekennzeichnet, daß die Kondensation der blockierten Produkte in Gegenwart von Dicyclohexylcarbodiimid in einem Lösungsmittel aus der Gruppe Dimethylformamid, Dimethylsulfoxid, Pyridin, Chloroform, Dichlormethan, Tetrahydrofuran und Aceton durchgeführt wird.

13. Verfahren gemäß Anspruch 8, dadurch gekennzeichnet, daß die Abspaltung der Schutzgruppen durch katalytische Hydrogenolyse oder kontrollierte Hydrolyse erfolgt.

14. Arzneimittel für human- oder veterinärmedizinische Zwecke, dadurch gekennzeichnet, daß sie enthalten oder bestehen aus einem oder mehreren Derivaten der nachstehenden allgemeinen Formel I:

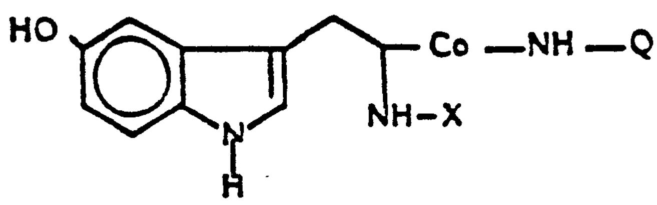

(I)

in der
X Wasserstoff oder ein niederes Acylradikal,
Q den Rest einer freien oder veresterten Aminosäure bedeutet,
sowie deren Additionssalze mit pharmazeutisch annehmbaren Säuren oder Basen.

## Claims

1. Dipeptides of -L-5-hydroxy-tryptophane of the following general formula I:

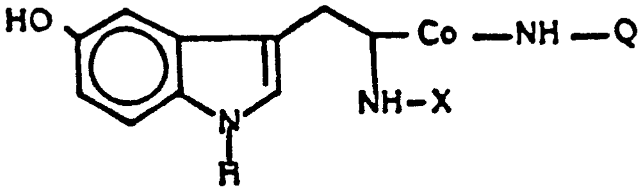

(I)

in which:
X represents hydrogen or a lower acyl radical,
Q represents a free amino acid residue, with the exception of tyrosine, or esterified, including here tyrosine, as well as their pharmaceutically compatible acid or basic addition salts.

2. Dipeptides according to Claim 1, in which Q represents a natural amino acid residue and X represents hydrogen or a lower acyl radical.

3. Dipeptides according to Claim 1, in which Q represents a non-natural amino acid residue and X represents hydrogen or a lower acyl radical.

4. Dipeptides according to any one of Claims 1 to 3, in which Q represents a dicarboxylic amino acid residue.

5. Dipeptides according to any one of Claims 1 to 3, in which Q represents a basic amino acid residue.

6. Dipeptides according to any one of Claims 1 to 3, in which Q represents an alcohol amino acid residue.

7. Dipeptides according to Claims 4, 5, 6, in which the reactive functions are substituted.

8. Process for the preparation of dipeptides according to Claims 1 to 7, characterized in that:
a) a derivative blocked at the nitrogen at the α position of the L-5-hydroxy-tryptophane is prepared,
b) a blocked derivative of an amino acid is prepared, allowing only the -$NH_2$ group to subsist as reactive function,
c) the two blocked products are condensed in the presence of a condensing agent ina suitable solvent at a temperature comprised between 0 and 30° C,
d) the protected functions are deblocked if necessary.

9. Process according to Claim 8, characterized in that the blocking groups at the nitrogen at the α position of the L-5-hydroxy-tryptophane (HTP) are selected from among the radicals carbobenzyloxy-, para-methoxy-benzyloxy-carbonyl, terbutyloxycarbonyl, the salts of DANE, with acetylacetone or ethyl acetoacetate.

10. Process according to Claim 8, characterized in that the blocking of the one or of both acid function of the amino acid is effected by means of compounds selected from the group which comprises primary, secondary or tertiary alcohol esters, benzyl esters.

11. Process according to Claim 8, characterized in that the L-5-hydroxy-tryptophane blocked at the nitrogen at the α position is used in the form of a reactive ester, said ester being selected from among the cyanomethyl, 4-nitrophenyl, and pentachlorophenyl esters.

12. Process according to Claim 8, characterized in that the condensation of the blocked products is carried out in the presence of dicyclohexylcarbodimide in a solvent taken from the group which comprises dimethylformamide, dimethylsulfoxide, pyridine, chloroform, dichloromethane, tetrahydrofurane and acetone.

13. Process according to Claim 8, characterized in that the unblocking of the protected functions is carried out by catalytic hydrogenation or controlled hydrolysis.

14. Medicaments for human or veterinary use, characterized in that they contain or are constituted by one or several derivatives corresponding to the following general formula I:

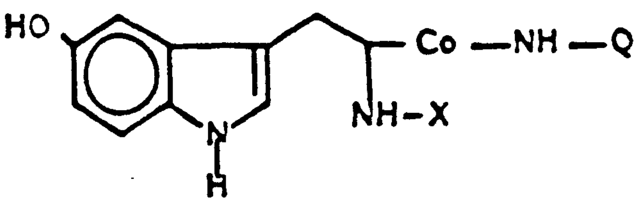

( I )

in which:
X respresents hydrogen or a lower acyl radical,
Q represents a free or esterified amino acid residue, as well as their pharmaceutically compatible acid or basic addition salts.